# EUROPEAN PATENT APPLICATION

(11) **EP 4 751 713 A1**
(43) Date of publication of application: **03.06.2026**
(21) Application number: 24845996.8
(22) Date of filing: 23.07.2024
(51) Int. Cl.: A61K 8/64, A61Q 19/00, A61Q 19/08, A61K 38/08, A61P 17/00, A23L 33/18

(54) **COMPOSITION FOR IMPROVING SKIN CONDITION COMPRISING PEPTIDE COMPLEX AS ACTIVE INGREDIENT**

(30) Priority: 27.07.2023 KR 20230098514
(71) Applicant: Caregen Co., Ltd., Seoul 06188 (KR)
(72) Inventor: CHUNG, Yong Ji, Seoul 06188 (KR); KIM, Eun Mi, Seoul 06188 (KR); LEE, Eung Ji, Seoul 06188 (KR)
(74) Representative: Handley, Matthew Edward
(86) International application number: PCT/KR2024/010596
(87) International publication number: WO 2025/023694

(57) **Abstract**

The present invention relates to a use of a peptide complex in improving skin condition. The peptide complex of the present invention has an activity of alleviating skin cell damage by inhibiting activation of inflammatory responses, promotion of apoptosis, increased expression of MMP-1 and MMP-2, increased expression of RAGE, and decreased expression of glyoxalase, which are various skin-damaging actions induced by advanced glycation end products or precursors thereof in skin cells.

## Description

### Technical Field

### [CROSS-REFERENCE TO RELATED APPLICATION]

This application claims the benefit of priority based on Korean Patent Application No. 10-2023-0098514 filed on July 27, 2023, and all contents disclosed in the relevant Korean Patent Application are included as part of the present specification.

### [Technical field]

The present disclosure relates to a use of a peptide complex for improving a skin condition.

### Background Art

The skin is an organ that covers all organs and tissues of the human body, protects the body from external invasion, and plays an important role in maintaining homeostasis by preserving moisture and regulating body temperature. Skin aging occurs naturally with increasing age and is also induced by external factors such as ultraviolet radiation and environmental pollution.

Meanwhile, advanced glycation end products (AGEs) are formed through non-enzymatic reactions between reducing sugars, such as glucose, and proteins under hyperglycemic conditions. Specifically, the carbonyl group of a reducing sugar reacts with free amino groups of proteins, such as lysine or arginine, to form an initial glycation product known as a Schiff base. As the reaction progresses, Amadori products are formed, and the resulting compounds subsequently undergo condensation, rearrangement, oxidation, fragmentation, and crosslinking processes to produce stable and irreversible compounds known as advanced glycation end products (AGEs).

The generated AGEs are known to be difficult to degrade in vivo and to damage organs or cells while remaining undegraded. In skin tissue, AGEs bind to collagen and elastin in the dermis, thereby inhibiting protein turnover, inducing tissue stiffening, and reducing elasticity, which contributes to wrinkle formation. In addition, when AGEs bind to the receptor for advanced glycation end products (RAGE) present on the surface of skin cells such as keratinocytes and fibroblasts, they are known to induce skin cell damage through the generation of reactive oxygen species (ROS), inflammatory responses, and activation of apoptosis signaling pathways (Chen C-Y et al., 2022, Advanced Glycation End Products in the Skin: Molecular Mechanisms, Methods of Measurement, and Inhibitory Pathways, Front. Med. 9:837222. doi:10.3389/fmed.2022.837222).

Synthetic pharmaceutical agents developed as inhibitors of AGE formation include aminoguanidine and pyridoxamine (Diabetes, 2002 Sep; 51(9):2826-32). However, these agents have been reported to induce toxicity upon long-term administration, thereby raising concerns regarding their safety and efficacy when applied to the skin. In addition, various technologies related to natural product-derived extracts having AGE-inhibitory activity and capable of improving skin damage have been developed (WO 2020/071630). However, such natural product-derived extracts often fail to exhibit sufficient activity and efficacy, making it difficult to develop them into final products.

Accordingly, there is a need to develop an active material that is safe and exhibits excellent activity for improving skin damage caused not only by various external factors but also by advanced glycation end products and their precursors.

### [Prior Art]

### [Patent document]

WO 2020/071630

### [Non-patent document]

1. Diabetes 2002 Sep; 51(9):2826-32
2. Front. Med. May 2022, Vol. 9:837222.

### Disclosure of the Invention

### Technical Problem

The present inventors had studied and made attempts to develop an active material to improve a skin condition by preventing skin damage caused by AGEs (advanced glycation end products), and experimentally proved that a peptide complex according to the present disclosure inhibits various cell damage actions caused by the AGEs in a skin cell and increases the expression of glyoxalase, which is involved in the removal of the AGEs, and to exhibit an activity for improving the skin condition.

Accordingly, an aspect of the present disclosure provides a composition for improving a skin condition.

Another aspect of the present disclosure provides a pharmaceutical composition for treating or preventing skin damage or skin diseases.

Another aspect of the present disclosure provides a functional food composition for preventing or improving skin damage.

### Technical Solution

To accomplish the objectives,
an aspect of the present disclosure provides a composition for improving a skin condition, which comprises, as an active ingredient, a peptide complex comprising: (i) a peptide comprising an amino acid sequence of SEQ ID NO. 1; and (ii) a peptide comprising an amino acid sequence of SEQ ID NO. 2.

Another aspect of the present disclosure provides a cosmetic composition for improving a skin condition comprising a peptide complex as an active ingredient.

Still another aspect of the present disclosure provides a pharmaceutical composition for treating or preventing skin inflammation or skin diseases, comprising a therapeutically effective amount of the peptide complex.

Still another aspect of the present disclosure provides a functional food composition for improve a skin condition comprising the peptide complex as an active ingredient.

Hereinafter, the details of the present disclosure will be described.

### Peptide complex and activity thereof

The present disclosure relates to the use for improving a skin condition of the peptide complex including (i) a peptide including the amino acid sequence of SEQ ID NO. 1; and (ii) a peptide including an amino acid sequence of SEQ ID NO. 2.

The peptide according to the present disclosure may refer to a linear molecule formed by binding amino acid residues to each other by a peptide bond.

According to the present disclosure, the peptide including the amino acid sequence of SEQ ID NO. 1 and the peptide including the amino acid sequence of SEQ ID NO. 2 may be used without modification. However, the variants or fragments of the amino acids may be used with different sequences, which are obtained through the deletion, insertion, or substitution of amino acid residues, or a combination thereof, as long as the variants or fragments do not affect the original activity of the peptide, for example, the activity of improving skin damage.

The peptide according to the present disclosure may be modified by phosphorylation, sulfation, acrylation, glycosylation, methylation, farnesylation, and the like as long as the activity of the peptide is not changed.

The peptide according to the present disclosure includes a peptide including an amino acid sequence substantially the same as that of a peptide including an amino acid sequence of SEQ ID NO. 1 or a peptide including the amino acid sequence of SEQ ID NO. 2, and a variant thereof or an active fragment thereof. The substantially same amino acid sequence may refer to an amino acid sequence having at least 75%, for example, at least 80%, at least 85%, at least 90%, at least 95%, at least 97%, or at least 99% sequence identical to the amino acid sequence of SEQ ID NO. 1 or the amino acid sequence of SEQ ID NO. 2, respectively. In addition, the peptide may further include an amino acid sequence which is designed for a specific purpose, such as a targeting sequence, a tag, labeled residues, or a sequence which increases the half-life or stability of the peptide.

The peptide of the present invention may be subjected to N-terminal and/or C-terminal modification to enhance its activity by selecting a portion of the amino acid sequence. The stability of the peptide according to the present disclosure may be significantly improved through the N-terminal and/or C-terminal modification. For example, the half-life may be increased when the peptide is administered in vivo. The term "stability" refers to not only stability in vivo to protect the peptide according to the present disclosure from attack of in vivo protein cleaving enzyme, but also storage stability (e.g., storage stability at a room temperature).

The N-terminal modification may be formed as a protecting group selected from the group consisting of an acetyl group, a fluorenylmethoxycarbonyl group, a formyl group, a palmitoyl group, a myristyl group, a stearyl group, and polyethylene glycol (PEG) is bonded to the N-terminus of the peptide. The C-terminal modification may be formed, as a hydroxyl group (-OH), or an amino group (-NH2), an azide is bonded to the C-terminus of the peptide, but the present disclosure is not limited thereto.

The peptide according to the present disclosure may be manufactured through various manners widely known in the art to which the present disclosure pertains. For example, the peptide according to the present disclosure is a chemical synthesis manner known in the art, particularly solid-phase synthesis techniques (Merrifield, J Amer. Chem. Soc. 85: 2149-54(1963); Stewart, et al. Solid Phase Peptide Synthesis, 2nd. Ed. Pierce Chem. Co: Rockford, 111(1984)) or a liquid synthesis technology (US Pat. No. 5,516,891).

The peptide complex according to the present disclosure has antiinflammatory activity in a skin cell.

The peptide complex according to the present disclosure may inhibit the activation of inflammatory responses induced by the AGEs or a precursor (methylglyoxal (MGO)) of the AGEs in the skin cell.

The peptide complex according to the present disclosure may inhibit the expression of inflammatory cytokines (TNF-α, IL-1 β, or IL-6) and an inflammation regulator COX-2 in the skin cell.

The peptide complex according to the present disclosure may inhibit the increase in the expression levels of TNF-α, IL-1 β, IL-6, and COX-2 induced by the AGEs and the precursor of the AGEs.

The peptide complex according to the present disclosure has an activity for inhibiting cell apoptosis in the skin cell.

The peptide complex according to the present disclosure may inhibit the cell apoptosis induced by the AGEs and the precursor of the AGEs.

The peptide complex according to the present disclosure may inhibit the expression of the cleaved PARP-1, the cleaved caspase-3, the cleaved caspase-9, or apoptosis-mediated protein Bax in the skin cell, and may increase the expression of anti-apoptotic protein Bcl-2.

The peptide complex according to the present disclosure may inhibit increased expression of cleaved PARP-1, increased cleaved caspase-3 expression, increased expression of cleaved caspase-9, and increased Bax expression induced by the AGEs or the precursor thereof in a skin cell.

The peptide complex according to the present disclosure may inhibit the decrease in the expression of anti-apoptotic protein (Bcl-2) induced by the AGEs and precursor of the AGEs in the skin cell.

The peptide complex according to the present disclosure has an activity for inhibiting the expression of matrix metalloproteinase (MMPs) in skin cells.

The peptide complex according to the present disclosure may inhibit the increase in the expression of matrix metalloproteinase (MMPs) induced by the AGEs and precursor of the AGEs in the skin cell.

The peptide complex according to the present disclosure may inhibit the increase in protein expression levels of MMP-1 and MMP-2 induced by the AGEs and precursor of the AGEs in the skin cell.

MMPs are proteolytic enzymes that specifically act on collagen. Representative MMPs include MMP-1 and MMP-2. The MMP-1 and MMP-2 degrade collagen, to reduce the elasticity of the skin tissue and the wrinkle production.

The peptide complex according to the present disclosure has the activity for inhibiting the expression of RAGE in the skin cell.

The peptide complex according to the present disclosure may inhibit an increase in the expression level of the RAGE induced by the AGEs and precursor of the AGEs in the skin cell.

The AGEs, which bind to the AGEs receptor present on the cell surface of the skin cells, generate ROS, induce inflammatory response, and activate the apoptosis signaling pathways through the AGEs receptor, thereby inducing skin cell damage.

The peptide complex according to the present disclosure inhibits the ROS generation, the induction of inflammatory response or the activation of cell apoptosis which is made through the RAGE.

The peptide complex according to the present disclosure has an activity of increasing the expression of glyoxalase-1 in skin cells.

The peptide complex according to the present disclosure inhibits the decrease in the expression level of glyoxalase-1 induced by the AGEs precursor. Accordingly, the peptide complex has the activity of increasing the expression of glyoxalase-1.

Glyoxalase-1 is a protein having the activity of degrading methylglyoxal, which is the AGEs precursor, and increases the expression of glyoxalase-1, thereby inhibiting the production of the AGEs.

### Composition for Improving Skin condition

An aspect of the present disclosure provides a composition for improving a skin condition, which includes, as an active ingredient, a peptide complex including: (i) a peptide including the amino acid sequence of SEQ ID NO. 1; and (ii) a peptide including an amino acid sequence of SEQ ID NO. 2.

The peptide complex according to the present disclosure having the above activity may exhibit the excellent effect for improving the skin condition.

According to an embodiment, the improvement of the skin condition by the composition for improving skin condition may refer to alleviation of skin inflammation, improvement of skin aging, promotion of skin regeneration, improvement of skin elasticity, alleviation of skin wrinkles, improvement of skin barriers, or improvement of skin damage.

According to an embodiment of the present disclosure, the improvement of the skin condition may refer to the improvement of the skin condition damaged by the AGEs and precursor of the AGEs.

In the composition of the present disclosure, the weight ratio between the peptide including the amino acid sequence of SEQ ID NO. 1 and the peptide including the amino acid sequence of SEQ ID NO. 2 is not particularly limited. For example, the weight ratio may include the weight ratio ranging from 0.5:2 to 2:0.5, and may include the weight ratio ranging from 0.5: 1.5 to 1.5: 0.5. Specifically, the weight ratio may include the weight ratio of 1:1.

According to an embodiment, the composition for improving skin condition according to the present disclosure has at least one of the following activities (a) to (e):
(a) Inhibition of expression of TNF-α, IL-1 β, or IL-6, or inhibition of expression of COX-2;
(b) Inhibition of the expression of cleaved PARP-1, cleaved caspase-3, cleaved caspase-9 or Bax, or increase in expression of Bcl-2;
(c) Inhibition of expression of MMP-1 or MMP-2;
(d) Inhibition of expression of Receptor Advanced Glycation End Products (RAGE); and
(e) Increase in expression of Glyoxalase-1.

The composition according to the present disclosure has antiinflammatory activity in a skin cell.

The composition according to the present disclosure may inhibit the activation of inflammatory responses induced by the AGEs or a precursor (MGO, methylglyoxal) of the AGEs in the skin cell.

The composition according to the present disclosure may inhibit the expression of inflammatory cytokines TNF-α, IL-1 β, or IL-6 and an inflammation regulator COX-2 in the skin cell.

The composition according to the present disclosure may inhibit the increase in the expression levels of TNF-α, IL-1 β, IL-6, and COX-2 induced by the AGEs and the precursor of the AGEs.

The composition according to the present disclosure has an activity for inhibiting cell apoptosis in the skin cell.

The composition according to the present disclosure may inhibit the cell apoptosis induced by the AGEs and the precursor of the AGEs in skin cell.

The composition according to the present disclosure may inhibit the expression of the cleaved PARP-1, the cleaved caspase-3, the cleaved caspase-9, or apoptosis-mediated protein Bax in the skin cell, and may increase the expression of anti-apoptotic protein Bcl-2 in skin cell.

The composition according to the present disclosure may inhibit increased expression of cleaved PARP-1, increased cleaved caspase-3 expression, increased expression of cleaved caspase-9, and increased Bax expression induced by the AGEs or the precursor thereof in a skin cell.

The composition according to the present disclosure may inhibit the decrease in the expression of anti-apoptotic protein (Bcl-2) induced by the AGEs and precursor of the AGEs in the skin cell.

The composition according to the present disclosure has an activity for inhibiting the expression of matrix metalloproteinase (MMPs) in skin cells.

The composition according to the present disclosure may inhibit the increase in the expression of matrix metalloproteinase (MMPs) induced by the AGEs and precursor of the AGEs in the skin cell.

The composition according to the present disclosure may inhibit the increase in protein expression levels of MMP-1 and MMP-2 induced by the AGEs and precursor of the AGEs in the skin cell.

MMPs are proteolytic enzymes that specifically act on collagen. Representative MMPs include MMP-1 and MMP-2. The MMP-1 and MMP-2 degrade collagen, to reduce the elasticity of the skin tissue and the wrinkle production.

The composition according to the present disclosure has the activity for inhibiting the expression of RAGE in the skin cell.

The composition according to the present disclosure may inhibit an increase in the expression level of the RAGE induced by the AGEs and precursor of the AGEs in the skin cell.

The AGEs bind to the AGEs receptor present on the cell surface of skin cells to generate ROS, induce inflammatory responses, and activate apoptosis signaling pathways, thereby inducing skin cell damage.

The peptide complex according to the present disclosure inhibits the ROS generation, the induction of inflammatory response or the activation of cell apoptosis which is made through the RAGE.

The peptide complex according to the present disclosure has an activity of increasing the expression of glyoxalase-1 in skin cells.

The peptide complex according to the present disclosure inhibits the decrease in the expression level of glyoxalase-1 induced by the AGEs precursor. Accordingly, the peptide complex has the activity of increasing the expression of glyoxalase-1.

Glyoxalase-1 is a protein having the activity of degrading methylglyoxal, which is the AGEs precursor, and increases the expression of glyoxalase-1, thereby inhibiting the production of the AGEs.

The composition according to the present disclosure may exhibit an effect for improving the skin condition.

According to the present disclosure, the composition for improving the skin condition may include a peptide complex at a dose ranging from 0.01 µM to 1,000 µM. Specifically, the peptide complex according to the present disclosure may be included at a dose ranging from 0.01 µM to 1,000 µM, a range from 0.05 µM to 800 µM, a range from 0.05 µM to 700 µM, a range from 0.05 µM to 600 µM, a range from 0.05 µM to 500 µM, a range from 0.05 µM to 300 µM, a range from 0.05 µM to 200 µM, a range from 0.1 µM to 800 µM, a range from 0.1 µM to 700 µM, a range from 0.1 µM to 600 µM, a range from 0.1 µM to 500 µM, a range from 0.1 µM to 300 µM, a range from 0.1 µM to 200 µM, a range from 1 µM to 800 µM, a range from 1 µM to 700 µM, a range from 1 µM to 600 µM, a range from 1 µM to 500 µM, a range from 1 µM to 300 µM, a range from 1 µM to 200 µM, a range from 5 µM to 800 µM, a range from 5 µM to 700 µM, a range from 5 µM to 600 µM, a range from 5 µM to 500 µM, a range from 5 µM to 300 µM, or a range from 5 µM to 200 µM, but the present disclosure is not limited thereto.

According to an embodiment, a composition for improving the skin condition may be a cosmetic composition.

The cosmetic composition may be prepared in any formulation commonly prepared in the art to which the present disclosure pertains, and may be an externally-applied dermal preparation. For example, the solution may be formulated into a solution, a suspension, an emulsion, a paste, a gel, a cream, a lotion, a powder, soap, a surfactant-containing cleansing, oil, a powder foundation, an emulsion foundation, a wax foundation, a spray, or the like, but the present disclosure is not limited thereto.

The cosmetic composition may be prepared in various forms such as a solution such as a softening lotion, a nourishing lotion, a nourishing cream, a massage cream, an essence, an eye cream, a cleansing cream, a cleansing foam, a cleansing water, a pack, a spray, a powder, a hair tonic, a hair cream, a hair lotion, a hair shampoo, a hair rinse, a hair conditioner, a hair spray, a hair aerosol, a pomade, and a gel, a sol-gel, an emulsion, an oil, a wax, an aerosol, and the like, but the present disclosure is not limited thereto.

The cosmetic composition according to the present disclosure may include other additives, such as excipients, or carriers. Accordingly, conventional ingredients commonly used in general skin cosmetic products may be incorporated in appropriate amounts.

When the form of the formulation of the cosmetic composition is paste, cream, or gel, animal oil, vegetable oil, wax, paraffin, starch, tragacanth, cellulose derivative, polyethylene glycol, silicone, bentonite, silica, talc, or zinc oxide may be used as the carrier component.

When the form of the formulation of the cosmetic composition is powder or spray, lactose, talc, silica, aluminum hydroxide, calcium silicate or polyamide powder may be used as a carrier component. In particular, when the form of the formulation of the cosmetic composition is the spray a propellant, such as chlorofluorohydrocarbon, propane/butane or dimethyl ether may be additionally included, but the present disclosure is not limited thereto.

When the form of the formulation of the cosmetic composition is a solution or an emulsion, a solvent, a solubilizer, or an emulsifying agent may be used as a carrier component. For example, water, ethanol, isopropanol, ethyl carbonate, ethyl acetate, benzyl alcohol, benzyl benzoate, propylene glycol, 1,3-butyl glycol oil, glycerol aliphatic ester, polyethylene glycol, fatty acid ester of sorbitan, and the like may be used.

When the formulation of the cosmetic composition is a suspension, a liquid diluent such as water, ethanol or propylene glycol, a suspending agent such as ethoxylated isostearyl alcohol, polyoxyethyl sorbitol ester and polyoxyethylene sorbitan ester, microcrystalline cellulose, aluminum metahydroxide, bentonite, agar, or tragacanth may be used as a carrier component.

When the formulation of the cosmetic composition is a surfactant-containing cleansing, aliphatic alcohol sulfate, aliphatic alcohol ether sulfate, sulfosuccinic acid monoester, isethionate, imidazolinium derivative, methyl taurate, sarcosinate, fatty acid amide ether sulfate, alkylamidobetaine, aliphatic alcohol, fatty acid glyceride, fatty acid diethanolamide, vegetable oil, lanolin derivative, ethoxylated glycerol fatty acid ester, or the like may be used as the carrier component.

When the formulation of the cosmetic composition is hair shampoo, base components for creating a shampoo, such as a thickener, a surfactant, a viscosity modifier, a moisturizer, a pH adjuster, a preservative, and an essential oil, may be mixed. As the thickener, CDE may be used, as the surfactant, LES, which is an anionic surfactant, and coco betaine, which is an amphoteric surfactant, may be used, and as the viscosity modifier, a poly quarter(polyquaternium) may be used, glycerin may be used as the moisturizer, and citric acid and sodium hydroxide may be used as the pH adjuster. As the preservative, a grapefruit extract may be used. In addition, essential oils such as cedarwood, peppermint and rosemary, silk amino acids, pentaol, or vitamin E may be added.

The components included in the cosmetic composition may further include a component commonly used in the cosmetic composition in addition to the peptide complex and the carrier component according to the present disclosure as active ingredients. For example, the components may further include conventional excipients, such as an antioxidant, a stabilizer, a solubilizer, vitamins, pigments, and flavorings, but the present disclosure is not limited thereto.

### Pharmaceutical composition

An aspect of the present disclosure provides a pharmaceutical composition for treating or preventing skin inflammation or skin diseases, which includes, as an active ingredient, a peptide complex including: (i) a peptide including the amino acid sequence of SEQ ID NO. 1; and (ii) a peptide including an amino acid sequence of SEQ ID NO. 2.

The peptide complex according to the present disclosure having the above activity may exhibit the excellent effect for treating or preventing skin inflammation or skin diseases.

According to the present disclosure, a pharmaceutical composition for treating or preventing skin inflammation or skin diseases has at least one activity of (a) to (e).
(a) Inhibition of expression of TNF-α, IL-1 β, or IL-6, or inhibition of expression of COX-2;
(b) Inhibition of the expression of cleaved PARP-1, cleaved caspase-3, cleaved caspase-9 or Bax, or increase in expression of Bcl-2;
(c) Inhibition of expression of MMP-1 or MMP-2;
(d) Inhibition of expression of Receptor Advanced Glycation End Products (RAGE); and
(e) Increase in expression of Glyoxalase-1.

The activities of the pharmaceutical composition of the present disclosure related to skin inflammation or skin diseases are the same as those described above for the composition for improving a skin condition, and thus, the description thereof is incorporated herein by reference and omitted to avoid redundancy.

The skin inflammation in the pharmaceutical composition may be skin inflammation caused by AGEs.

The skin disease of the pharmaceutical composition may be AGEs-related skin disease.

According to the present disclosure, the AGEs-related skin disease refers to skin disease directly or indirectly caused by the AGEs.

The AGEs-related skin disease may be bacterial skin infections, skin wounds, diabetic skin wounds, diabetic dermatopathy, diabetic foot disease, hyperpigmentation disorders, photo-induced hyperpigmentation disorders, psoriasis, or systemic lupus erythematosus, and the bacterial skin infections may be skin infections of Staphylococcus aureus, and the hyperpigmentation disorder may be a photo-induced hyperpigmentation disorder, but the present disclosure is not limited thereto.

The contents of the AGEs-related skin diseases are described in Manli Na et al. The Journal of Infectious Diseases, 218(5): 791-800(2018); Hwan June Kang et al. Journal of Controlled Release 333: 176-187(2021); Jiaqi Fang et al. Journal of Investigative Dermatology, 142 (10): 2591-2602 (2022); Anastasia Papagracoraki et al. Int. J Mol. 18, 2471 (2017); Nowak, A et al. Cells, 10, 523(2021); Lee et al. Kor. J Herbal. 32(5) 7-12 (2017); Kim et al. Kor. J Herbal. 32(4) 1717-24 (2017), which are incorporated herein by reference.

As used herein, the term "preventing" refers to reducing the risk of skin inflammation or skin disease, and refers to all actions of inhibiting or delaying the onset of a disease by preventing the skin inflammation or skin disease or at least one clinical symptom thereof from proceeding.

As used herein, the term "treating" refers to alleviating skin inflammation or skin diseases, and includes all actions of reducing or decreasing the progression of at least one clinical symptom of the skin inflammation or skin disease to cure or change the symptoms of the disease.

The pharmaceutical composition according to the present disclosure may include a therapeutically effective amount of the peptide complex.

The term "therapeutically effective amount" refers to an amount sufficient to achieve the activity or efficacy of the peptide complex, which is an active ingredient of the pharmaceutical composition of the present disclosure, and refers to, for example, an amount sufficient to achieve the efficacy of treating or preventing skin inflammation or skin diseases.

The pharmaceutical composition according to the present disclosure may include a pharmaceutically acceptable carrier.

Examples of the pharmaceutically acceptable carrier include lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, and the like, but the present disclosure is not limited thereto.

The pharmaceutical composition may be prepared in a unit dosage form or inserted into a multi-dose container by using a pharmaceutically acceptable carrier and/or excipient according to a method which may be easily performed by those skilled in the art to which the present disclosure pertains. In this case, the formulation may be in the form of a solution, suspension, or emulsion in an oil or aqueous medium, or in the form of an extract, a powder agent, a granule agent, a tablet agent, a capsule agent, or gel (e.g. hydrogel), and may additionally include a dispersant or a stabilizer.

The pharmaceutical composition according to the present disclosure may further include, in addition to the above components, a lubricant, a wetting agent, a sweetener, a flavoring agent, an emulsifier, a suspending agent, or a preservative, but the present disclosure is not limited thereto.

Suitable pharmaceutically acceptable carriers and formulations are described in detail in Remington's Pharmaceutical Sciences (19th ed; 1995).

The pharmaceutical composition according to the present disclosure may be administered in all suitable routes for treating skin inflammation or skin diseases, and for example, may be administered orally or parenterally, and when administered parenterally, the pharmaceutical composition may be administered by intravenous injection, subcutaneous injection, intramuscular injection, intraperitoneal injection, topical administration, transdermal administration, or the like.

The dosage of the pharmaceutical composition may be a range from 0.0001 µg to 100 mg, a range from 0.001 µg to 100 mg, a range from 0.01 µg to 100 mg, a range from 0.1 µg to 100 mg, or a range from 1.0 µg to 1000 mg per day, but the present disclosure is not limited thereto. The dosage of the pharmaceutical composition may be variously prescribed by factors such as a formulation manner, administration manner, patient age, weight, sex, pathological condition, food, administration time, administration route, excretion rate, and response sensitivity.

The pharmaceutical composition according to the present disclosure may be prepared in a unit dosage form or inserted into a multi-dose container by using a pharmaceutically acceptable carrier and/or excipient according to a manner which may be easily performed by those skilled in the art to which the present disclosure pertains. In this case, the formulation may be in the form of a solution, suspension, or emulsion in an oil or aqueous medium, or in the form of an X-agent, a powder agent, a granule agent, a tablet agent, a capsule agent, or gel (e.g. hydrogel), and may additionally include a dispersant or a stabilizer.

The pharmaceutical composition according to the present disclosure may be used alone or in combination with manners using surgery, radiotherapy, hormone therapy, chemotherapy, and biological response modifiers to treat or prevent skin inflammation or skin diseases.

The pharmaceutical composition according to the present disclosure may be an externally-applied dermal preparation. The externally-applied dermal preparation is a formulation that may be applied to the outside of the skin. When the pharmaceutical composition according to the present disclosure is used as the externally-applied dermal preparation, the pharmaceutical composition may be applied to the skin, specifically, a skin site where skin inflammation or skin diseases occur. The externally-applied dermal preparation may be a cream, a gel, an ointment, a skin emulsifier, a skin suspension, a transdermal patch, a drug-containing bandage, a lotion, or a combination thereof. The externally-applied dermal preparation may include externally-applied dermal preparation for cosmetics or medicines. For example, the externally-applied dermal preparation may include an aqueous component, an oily component, a powder component, an alcohol, a moisturizer, a medium-viscosity agent, an ultraviolet absorber, a whitening agent, a preservative, an antioxidant, a surfactant, a fragrance, a colorant, various skin nutrients, or a combination thereof, and a material formulated by appropriately mixing the above materials. The externally-applied dermal preparation may be formulated by appropriately mixing a metal chelating agent, such as disodium edetate, trisodium edetate, sodium citrate, sodium polyphosphate, sodium methanate, or gluconate, a hot water extract, such as caffeine, tannins, bellafamil, licorice extract, glabridine, and caline fruits, various herbal medicines, acetic acid tocopherol, glycidylic acid, tranexamic acid, derivatives of the tranexamic acid, or medicine such as a salt of the tranexamic acid, saccharides such as vitamin C, magnesium ascorbyl phosphate, ascorbyl glucoside, arbutin, or kojic acid, sugars such as glucose, fructose, and trehalose.

### Food composition

Another aspect of the present disclosure provides a functional food composition for improving a skin condition, which includes, as an active ingredient, a peptide complex including: (i) a peptide including the amino acid sequence of SEQ ID NO. 1; and (ii) a peptide including an amino acid sequence of SEQ ID NO. 2.

The improvement of the skin condition may refer to alleviation of skin inflammation, improvement of skin aging, promotion of skin regeneration, improvement of skin elasticity, alleviation of skin wrinkles, improvement of skin barriers, or improvement of skin damage.

The activity related to the improvement of the skin condition in the functional food composition according to the present disclosure has been already described in the composition for the improvement of the skin condition described above. Accordingly, the details thereof are incorporated herein by reference and will be omitted to avoid redundancy.

In the functional food composition of the present disclosure, the peptide complex may be included by selecting an appropriate amount within a range of at most 10 wt %, specifically, a range from 0.000001 wt % to 10 wt %, based on the total weight of the composition.

The functional food composition according to the present disclosure may include a food-effective amount of a peptide complex and a food-acceptable carrier.

The functional food composition according to the present disclosure includes not only a peptide complex as an active ingredient, but also a component commonly added in the preparation of food, and may include, for example, a protein, a carbohydrate, a fat, a nutrient, a seasoning, and a flavoring agent. The above-described carbohydrate includes a monosaccharide, such as glucose, or fructose, a disaccharide, such as maltose, sucrose, and oligosaccharide, polysaccharide including a typical sugar, such as dextrin, and cyclodextrin, and sugar alcohol, such as xylitol, sorbitol, or erythritol. The flavoring agent may include a natural flavoring agent, thaumatin, and a stevia extract (e.g., rebaudioside A, glycyrrhizin), and a synthetic flavoring agent (e.g., saccharin and aspartame).

The functional food composition according to the present disclosure may include various nutrients, vitamins, minerals (electrolytes), flavoring agents such as synthetic flavors and natural flavors, colorants and enhancers (cheese, or chocolate), pectic acid and salts thereof, alginic acid and salts thereof, organic acids, protective colloidal thickeners, pH adjusters, stabilizers, preservatives, glycerin, alcohols, and carbonating agents used in carbonated beverages, in addition the above ingredients. In addition, the functional food composition may include pulp for preparing natural fruit juice and fruit juice beverages and vegetable beverages. For example, when the functional food composition according to the present disclosure is prepared as a drink agent, the functional food composition may include citric acid, liquid fructose, sugar, glucose, acetic acid, apple acid, fruit juice, eucommia extract, jujube extract, licorice extract, in addition to the peptide, which is the active ingredient of the present disclosure.

The functional food may be provided in various types without a particular limitation. The functional food and the foods may include, for example, meat, sausages, bread, chocolate, candies, snacks, biscuits, pizza, ramen, other noodles, gum, ice cream, dairy products, various soups, beverages, tea drinks, alcoholic beverages, vitamin complexes, dairy products, and fermented milk, and includes all typical functional foods or foods.

### Use of the Peptide complex according to the present disclosure

Another aspect of the present disclosure provides the use of a peptide complex including: (i) a peptide including the amino acid sequence of SEQ ID NO. 1; and (ii) a peptide including an amino acid sequence of SEQ ID NO. 2 to improve the skin condition, treat, prevent, or alleviate skin inflammation or skin diseases.

Another aspect of the present disclosure provides the use of a peptide complex including: (i) a peptide including the amino acid sequence of SEQ ID NO. 1; and (ii) a peptide including an amino acid sequence of SEQ ID NO. 2 to prepare a medicine, cosmetics, or health functional food for improving the skin condition, treating, preventing, or alleviating skin inflammation or skin diseases.

Another aspect of the present disclosure provides a method for improving a skin condition, or a method for treating, preventing, or alleviating skin inflammation or skin disease, which includes administering a peptide complex including: (i) a peptide including an amino acid sequence of SEQ ID NO. 1; and (ii) a peptide including an amino acid sequence of SEQ ID NO. 2 to a subject in need of improving of the skin condition or a subject in need of treating, preventing, or alleviating the skin inflammation or the skin diseases.

The subject may be a human being or an animal except for the human being. In addition, the subject may be a human being having skin inflammation or skin disease or an animal having skin inflammation or skin disease, except for the human being.

The description about the peptide complex, composition, cosmetics composition, pharmaceutical composition, and functional food composition according to the present disclosure is identically applicable to the method for improving the skin condition, and the method for treating, preventing, or alleviating skin inflammation or skin disease, and the redundant repetition thereof will be omitted to avoid excessive complexity of this specification.

### Advantageous Effects

The peptide complex according to the present disclosure has an activity for inhibiting the damage to the skin cell. The peptide complex according to the present disclosure inhibits various skin damage actions, such as the activation of inflammatory response, the promotion of cell apoptosis, the increase in the expression of MMP-1 and MMP-2, the increase in the expression of RAGE, and the decrease in the expression of Glyoxalase, which are induced by the AGEs or the AGEs precursor in skin cells, thereby improving the damage to skin cells. The effects of the present disclosure are not limited to the aforementioned effects, and any other effects not mentioned herein will be clearly understood from the following description by those skilled in the art to which the present disclosure pertains.

### Brief Description of the Drawings

FIG. 1A illustrates results of decreasing expression levels of TNF-α, IL-1 β, and IL-6, which are inflammatory response factors, increased through AGE-BSA treatment in HaCaT keratinocytes, through treatment of the peptide complex, and FIG. 1B is a graph obtained by quantifying the intensity of the band of FIG. 1A.
FIG. 2A illustrates results of decreasing expression levels of TNF-α, IL-1 β, and IL-6, which are inflammatory response factors, increased through MGO treatment in HaCaT keratinocytes, through treatment of the peptide complex, and FIG. 2B is a graph obtained by quantifying the intensity of the band of FIG. 2A.
FIG. 3A illustrates results of decreasing expression levels of COX-2, IL-1β, and IL-6, which are inflammatory response factors, increased through AGE-BSA treatment in NIH3T3 fibroblasts, through treatment of the peptide complex, and FIG. 3B is a graph obtained by quantifying the intensity of the band of FIG. 3A.
FIG. 4A illustrates results of decreasing expression levels of cleaved PARP-1, cleaved caspase-3, cleaved caspase-9, which are apoptosis-regulating factors, Bax, which is apoptosis-mediated protein, increased through AGE-BSA treatment in HaCaT keratinocytes, through treatment of the peptide complex, and FIG. 4B is a graph obtained by quantifying the intensity of the band of FIG. 4A.
FIG. 5A illustrates results showing that expression levels of cleaved caspase-3 and cleaved caspase-9, which are apoptosis-regulating factors, increased by MGO treatment in HaCaT keratinocytes, are decreased by treatment with the peptide complex, and that the expression level of Bcl-2, which is an anti-apoptotic protein, decreased by MGO treatment, is increased by treatment with the peptide complex. FIG. 5B is a graph obtained by quantifying the intensity of the band of FIG. 5A.
FIG. 6A illustrates results of decreasing expression levels of cleaved PARP-1, cleaved caspase-3, cleaved caspase-9, which are apoptosis-regulating factors, Bax, which is apoptosis-mediated protein, increased through AGE-BSA treatment in NIH3T3 fibroblasts, through treatment of the peptide complex, and results of increasing an expression level of Bcl-2, which is anti-apoptotic protein, decreased through the AGE-BSA treatment in NIH3T3 fibroblasts, through treatment of the peptide complex, and FIG. 6B is a graph obtained by quantifying the intensity of the band of FIG. 6A.
FIG. 7A illustrates results of decreasing expression levels of MMP-1 and MMP-2, increased through AGE-BSA treatment in HaCaT keratinocytes, through treatment of the peptide complex, and FIG. 7B is a graph obtained by quantifying the intensity of the band of FIG. 7A.
FIG. 8A illustrates results of decreasing expression levels of MMP-1 and MMP-2, increased through MGO treatment in HaCaT keratinocytes, through treatment of the peptide complex, and FIG. 8B is a graph obtained by quantifying the intensity of the band of FIG. 8A.
FIG. 9A illustrates results of decreasing expression levels of MMP-1, increased through AGE-BSA treatment in NIH3T3 fibroblasts, through treatment of the peptide complex, and FIG. 9B is a graph obtained by quantifying the intensity of the band of FIG. 9A.
FIG. 10A illustrates results of decreasing expression levels of RAGE, increased through AGE-BSA treatment in HaCaT keratinocytes, through treatment of the peptide complex, and FIG. 10B is a graph obtained by quantifying the intensity of the band of FIG. 10A.
FIG. 11A illustrates results of decreasing expression levels of RAGE, increased through MGO treatment in HaCaT keratinocytes, through treatment of the peptide complex, and FIG. 11B is a graph obtained by quantifying the intensity of the band of FIG. 11A.
FIG. 12A illustrates results of decreasing expression levels of RAGE, increased through AGE-BSA treatment in NIH3T3 fibroblasts, through treatment of the peptide complex, and FIG. 12B is a graph obtained by quantifying the intensity of the band of FIG. 12A.
FIG. 13A illustrates results of increasing expression levels of Glyoxalase-1, decreased through MGO treatment in HaCaT keratinocytes, through treatment of the peptide complex, and FIG. 13B is a graph obtained by quantifying the intensity of the band of FIG. 13A.

### Mode for Invention

Hereinafter, the present disclosure will be described in detail. However, the following example is provided only for the illustrative purpose of the present disclosure in more detail, and the present disclosure is not limited to the following description.

### Preparation example 1: Preparation of Peptide Complex

A peptide having the amino acid sequence of SEQ ID NO. 1 and a peptide having the amino acid sequence of SEQ ID NO. 2 shown in Table 1 below individually were synthesized using an automatic peptide synthesizer (Milligen 9050; Millipore; USA), and the synthesized peptides were purified and separated using C18 reverse-phase high performance liquid chromatography (HPLC) (Waters Associates, USA) A column employed was an ACQUITY UPLC BEH300 C18 (2.1 mm × 100 mm; 1.7 µm; Waters Co., USA).

**[Table 1]**

| Amino acid sequence | Sequence No. |
|---|---|
| KGSATGWMA | 1 |
| LKTRN | 2 |

Thereafter, a peptide complex was prepared by mixing the peptide of the amino acid sequence of SEQ ID NO. 1 and the peptide of the amino acid sequence of SEQ ID NO. 2 in the same amount, and the effect of the peptide complex prepared in the following Experimental example was evaluated.

### Experimental example 1: Measurement of expression level of inflammatory cytokine in skin cell

The effect of the peptide complex prepared in Preparation Example 1 on the expression levels of inflammatory cytokines induced by AGEs or methylglyoxal (MGO), a precursor thereof, in skin cells was evaluated. HaCaT human skin keratinocytes and NIH3T3 fibroblasts were seeded in 6-well plates at a density of 2 × 10⁵ cells per well and cultured for 24 hours. Thereafter, the cells were pretreated with the peptide complex prepared in Preparation Example 1 at concentrations of 50 µM, 100 µM, and 200 µM for 1 hour. The pretreated cells were treated with 200 µg/mL of AGE-BSA or 200 µM of methylglyoxal (MGO) and cultured in a CO₂ incubator at 37°C for 48 hours. In the AGE-BSA treatment experiment, control wells were treated with 200 µg/mL of BSA. After treatment, the cells were washed twice with PBS, and total RNA was isolated using easy-BLUETM (iNtRON, Cat. No.: 17061, Korea). The isolated total RNA was quantified, and cDNA was synthesized using an RT kit (enzynomics, Cat. No.: RT200, Korea). PCR was then performed using a PCR kit (enzynomics, Cat. No.: P581T, Korea). The primers used are shown in Table 2 below.

**[Table 2]**

| Cell line | Primer | Sequence (5 '-> 3 ') | Sequence No. |
|---|---|---|---|
| HaCaT | TNF-α Forward | AACATCCAACCTTCCCAAACG | 3 |
| | TNF-α Reverse | GACCCTAAGCCCCCAATTCTC | 4 |
| | IL-1β Forward | TTCGACACATGGGATAACGA | 5 |
| | IL-1β Reverse | TCTTTCAACACGCAGGACAG | 6 |
| | IL-6 Forward | AAAGAGGCACTGCCAGAAAA | 7 |
| | IL-6 Reverse | ATCTGAGGTGCCCATGCTAC | 8 |
| NIH3T3 | COX-2 Forward | TGAGTGGTAGCCAGCAAAGC | 9 |
| | COX-2 Reverse | CTGCAGTCCAGGTTCAATGG | 10 |
| | IL-1β Forward | CCACAGACCTTCCAGGAGAAT G | 11 |
| | IL-1β Reverse | GTGCAGTTCAGTGATCGTACA GG | 12 |
| | IL-6 Forward | CACGGCCTTCCCTACTTCAC | 13 |
| | IL-6 Reverse | AGCCACTCCTTCTGTGACTC | 14 |
| HaCaT/N IH3T3 | GAPDH Forward | GGAGCCAAAAGGGTCATCAT | 15 |
| | GAPDH Reverse | GTGATGGCATGGACTGTGGT | 16 |

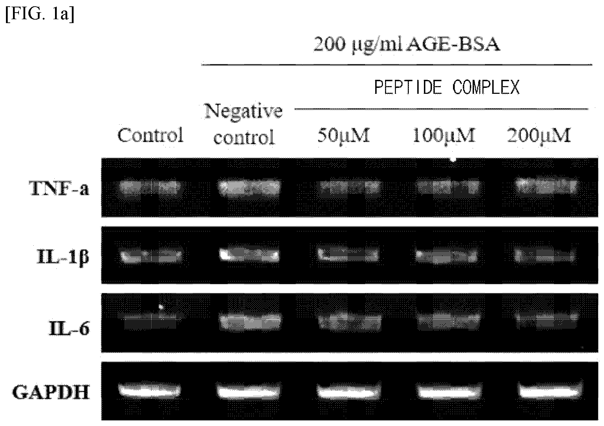

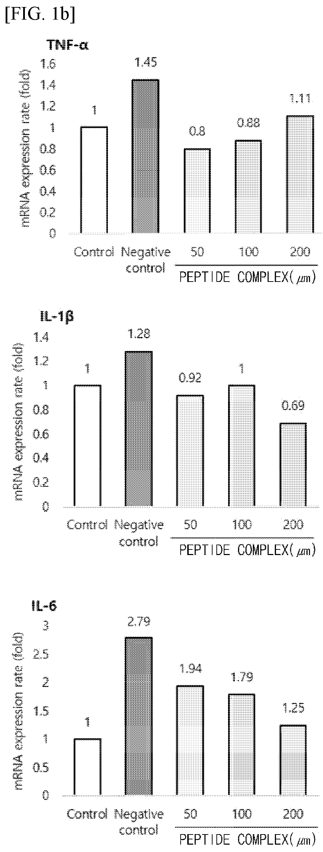

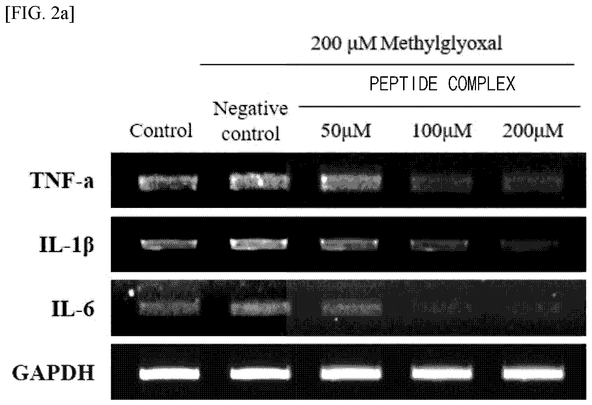

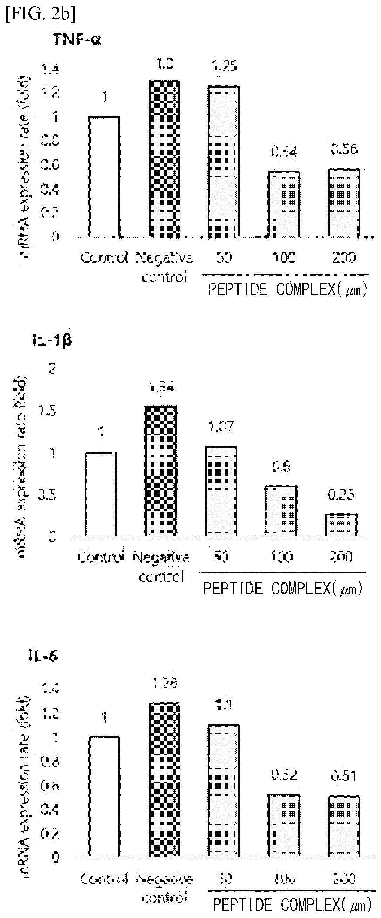

In HaCaT keratinocytes, the mRNA expression levels of inflammatory cytokines TNF-α, IL-1β, and IL-6, as markers of an inflammatory response induced by AGE-BSA, were measured, and the results are shown in FIGS. 1A and 1B. As shown in FIGS. 1A and 1B, the mRNA expression levels of TNF-α, IL-1β, and IL-6 were increased by AGE-BSA treatment in a negative control group as compared to a control group. Treatment with the peptide complex of the present invention reduced the mRNA expression levels of TNF-α, IL-1β, and IL-6 as compared to the negative control group. In addition, in HaCaT keratinocytes, the mRNA expression levels of inflammatory cytokines TNF-α, IL-1β, and IL-6, as markers of an inflammatory response induced by methylglyoxal (MGO), were measured, and the results are shown in FIGS. 2A and 2B. As shown in FIGS. 2A and 2B, the mRNA expression levels of TNF-α, IL-1β, and IL-6 were increased by MGO treatment in a negative control group as compared to a control group. Treatment with the peptide complex of the present invention reduced the mRNA expression levels of TNF-α, IL-1β, and IL-6 as compared to the negative control group.

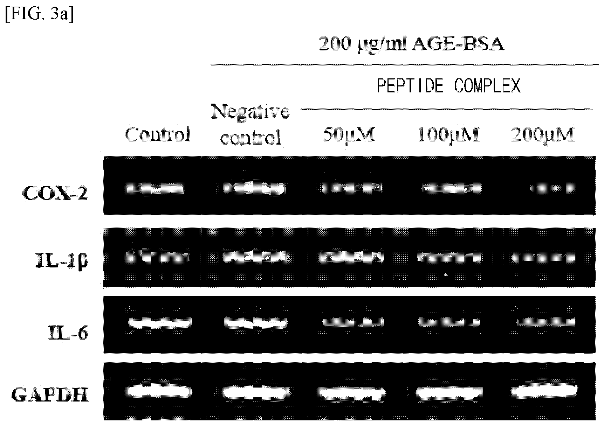

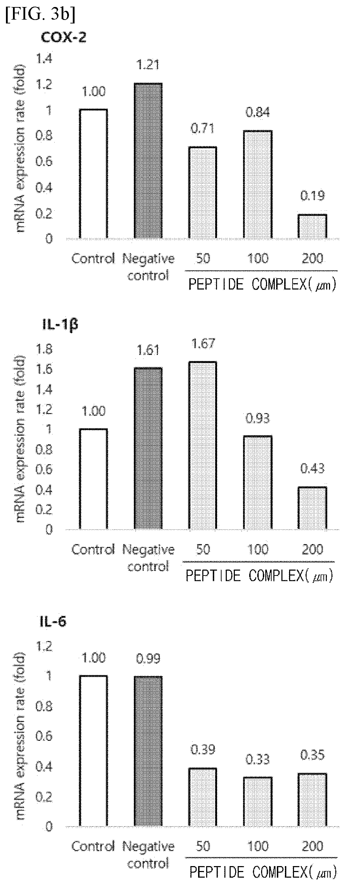

The mRNA expression levels of COX-2 (an inflammatory mediator), and IL-1β, and IL-6 (inflammatory cytokines), serving as markers of inflammation induced by AGE-BSA in NIH3T3 fibroblasts, were measured, and the measurement results were illustrated in FIGS. 3A and 3B. As illustrated in FIGS. 3A and 3B, the mRNA expression levels of COX-2 and IL-1β in the negative control group through AGE-BSA treatment was more increased as compared to the control group. As the peptide complex was treated according to the present disclosure, the mRNA expression levels of COX-2 and IL-6 were decreased as compared to the negative control group. For IL-1β, when the peptide complex was treated at the dose of 50 µM, the mRNA expression level was slightly higher as compared to the negative control group. However, when the peptide complex was treated at the doses of 100 µM and 200 µM, the mRNA expression level was decreased.

### Experimental example 2: Measurement of expression levels of apoptosis-regulating factors, MMPs, and RAGE in skin cells

### 1. Experimental method

To evaluate the skin damage-improving effect of the peptide complex prepared in Preparation Example 1, the expression levels of apoptosis-regulating factors, matrix metalloproteinases (MMPs), and receptor for advanced glycation end products (RAGE) in skin cells were examined by Western blotting.

Specifically, HaCaT human skin keratinocytes and NIH3T3 fibroblasts were seeded in 6-well plates at a density of 2 × 10⁵ cells per well and cultured for 24 hours. Thereafter, the cells were pretreated with the peptide complex prepared in Preparation Example 1 at concentrations of 50 µM, 100 µM, and 200 µM for 1 hour. The pretreated cells were then treated with 200 µg/mL of AGE-BSA or 200 µM of methylglyoxal (MGO) and cultured in a CO₂ incubator at 37°C for 48 hours. In the AGE-BSA treatment experiment, control wells were treated with 200 µg/mL of BSA. After treatment, the cells were washed twice with PBS and lysed by adding a cell lysis buffer. After treatment with a 5× sample buffer, SDS-PAGE was performed using a 10% SDS-PAGE gel, and the proteins separated by SDS-PAGE were transferred to a PVDF membrane. The membrane was blocked with 5% skim milk at room temperature for 1 hour. Each primary antibody listed in Table 3 below was diluted in 5% skim milk at a dilution ratio of 1:1000 and incubated with the membrane for 2 hours, followed by washing three times with 0.1% PBS-T (0.1% Tween-20 in PBS) for 10 minutes each. Thereafter, each secondary antibody corresponding to the respective primary antibody listed in Table 3 below was diluted in 5% skim milk at a dilution ratio of 1:3000 and incubated with the membrane for 2 hours. After treatment with an ECL solution (GE Healthcare, Cat. No.: RPN2232, USA), images were obtained using an ImageQuant 800 system (Cytiva, Cat. No.: 29-3994-81, USA).

**[Table 3]**

| **Primary antibody** | | | | **Secondary antibody** | | |
|---|---|---|---|---|---|---|
| **Cell line** | **Antibodies** | **Manufacturer** | **Cat. No.** | **Antibodies** | **Manufacturer** | **Cat. No.** |
| HaCaT | Cleaved PARP-1 | cell signaling technology (USA) | 9541s | Peroxidase-AffiniPure Goat Anti-Rabbit IgG (H+L) | Jackson ImmunoResearch (USA) | 111-035-003 |
| NIH3T3 | | Santa Cruz Biotechnology (USA) | sc56196 | Peroxidase-AffiniPure Goat Anti-Mouse IgG (H+L) | Jackson ImmunoResearch (USA) | 115-035-003 |
| HaCaT / NIH3T3 | Cleaved caspase-3 | abcam (UK) | ab184787 | Peroxidase-AffiniPure Goat Anti-Rabbit IgG (H+L) | Jackson ImmunoResearch (USA) | 111-035-003 |
| HaCaT / NIH3T3 | Cleaved caspase-9 | abcam (UK) | ab25758 | Peroxidase-AffiniPure Goat Anti-Rabbit IgG (H+L) | Jackson ImmunoResearch (USA) | 111-035-003 |
| HaCaT | Bax | Santa Cruz Biotechnology (USA) | sc20067 | Peroxidase-AffiniPure Goat Anti-Mouse IgG (H+L) | Jackson ImmunoResearch (USA) | 115-035-003 |
| NIH3T3 | | Santa Cruz Biotechnology (USA) | sc7480 | Peroxidase-AffiniPure Goat Anti-Mouse IgG (H+L) | Jackson ImmunoResearch (USA) | 115-035-003 |
| HaCaT / NIH3T3 | Bcl-2 | Santa Cruz Biotechnology (USA) | sc7382 | Peroxidase-AffiniPure Goat Anti-Mouse IgG (H+L) | Jackson ImmunoResearch (USA) | 115-035-003 |
| HaCaT / NIH3T3 | MMP-1 | abcam (UK) | ab137332 | Peroxidase-AffiniPure Goat Anti-Rabbit IgG (H+L) | Jackson ImmunoResearch (USA) | 111-035-003 |
| HaCaT / NIH3T3 | MMP-2 | abcam (UK) | ab86607 | Peroxidase-AffiniPure Goat Anti-Mouse IgG (H+L) | Jackson ImmunoResearch (USA) | 115-035-003 |
| HaCaT / NIH3T3 | RAGE | Santa Cruz Biotechnology (USA) | sc33662 | Peroxidase-AffiniPure Goat Anti-Mouse IgG (H+L) | Jackson ImmunoResearch (USA) | 115-035-003 |
| HaCaT / NIH3T3 | α-tubulin | Santa Cruz Biotechnology (USA) | sc69969 | Peroxidase-AffiniPure Goat Anti-Mouse IgG (H+L) | Jackson ImmunoResearch (USA) | 115-035-003 |

### 2. Observation of effect for inhibiting skin cell apoptosis

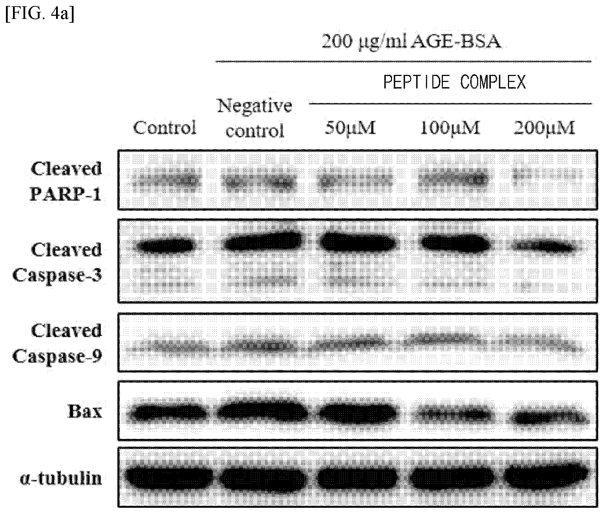

To evaluate the apoptosis-inhibitory effect of the peptide complex on skin cells, the expression levels of apoptosis-regulating factors were measured, and the results are shown in FIGS. 4A, 4B, 5A, 5B, 6A, and 6B. Cleaved PARP-1, cleaved caspase-3, and cleaved caspase-9 are characteristic marker proteins for apoptosis, Bax is a pro-apoptotic mediator protein, and Bcl-2 is an anti-apoptotic protein.

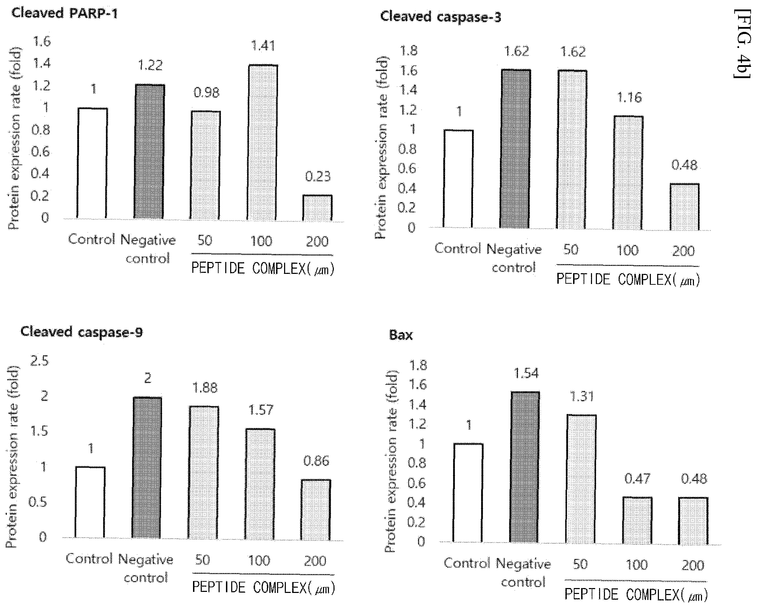

First, in HaCaT keratinocytes, the expression levels of cleaved PARP-1, cleaved caspase-3, cleaved caspase-9, and Bax, as apoptosis-regulating factors induced by AGE-BSA treatment, were measured, and the results are shown in FIGS. 4A and 4B. As shown in the negative control groups of FIGS. 4A and 4B, the expression levels of cleaved caspase-3, cleaved caspase-9, and Bax were increased by AGE-BSA treatment, and the increased expression levels of these apoptosis-regulating proteins were reduced by treatment with the peptide complex.

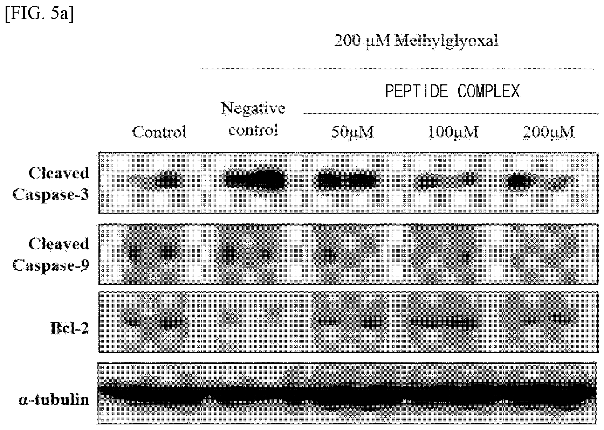

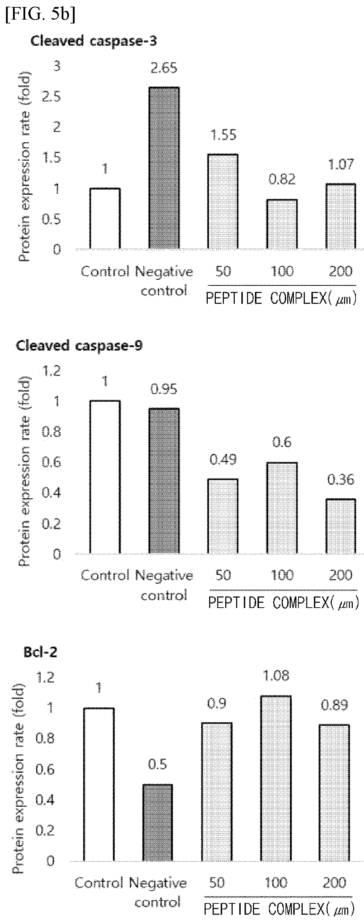

In addition, in HaCaT keratinocytes, the expression levels of cleaved caspase-3, cleaved caspase-9, and Bcl-2, as apoptosis-regulating factors whose expression levels were altered by MGO treatment, were measured, and the results are shown in FIGS. 5A and 5B. As shown in the negative control groups of FIGS. 5A and 5B, the expression level of cleaved caspase-3 was increased by MGO treatment, the expression level of cleaved caspase-9 was not changed, and the expression level of the anti-apoptotic factor Bcl-2 was decreased. However, as shown in the experimental groups, treatment with the peptide complex reduced the expression levels of cleaved caspase-3 and cleaved caspase-9. In addition, the expression level of Bcl-2, which was decreased by MGO treatment, was increased by treatment with the peptide complex.

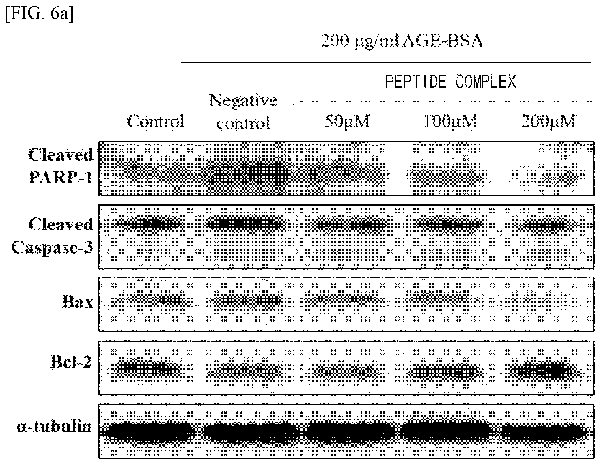

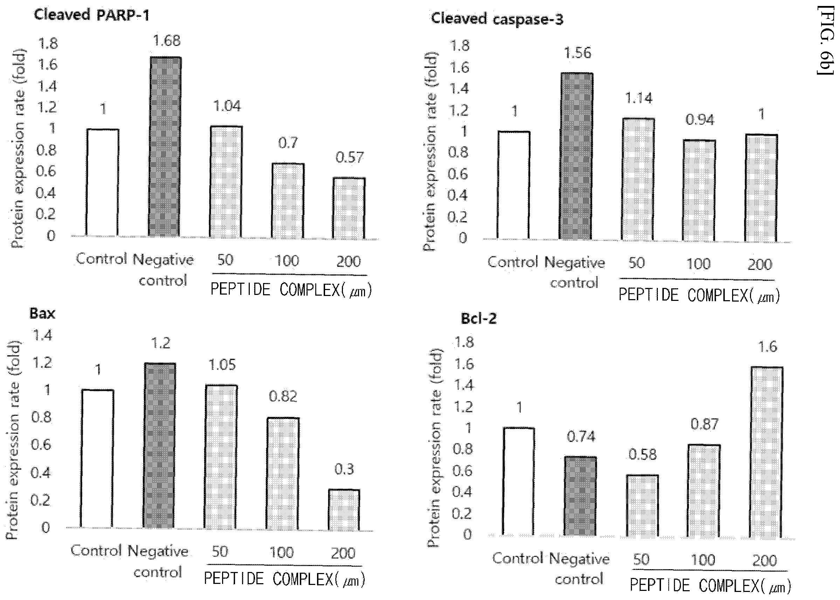

In NIH3T3 fibroblasts, the expression levels of cleaved PARP-1, cleaved caspase-3, cleaved caspase-9, Bax, and Bcl-2, as apoptosis-regulating factors whose expression levels were altered by AGE-BSA treatment, were measured, and the results are shown in FIGS. 6A and 6B. As shown in the negative control groups of FIGS. 6A and 6B, the expression levels of cleaved PARP-1, cleaved caspase-3, cleaved caspase-9, and Bax were increased by AGE-BSA treatment, whereas the expression level of the anti-apoptotic factor Bcl-2 was decreased. However, as shown in the experimental groups, the expression levels of cleaved PARP-1, cleaved caspase-3, and Bax, which were increased by AGE-BSA treatment, were reduced by treatment with the peptide complex. For Bcl-2, the expression level, which was reduced in the negative control group, was increased in a dose-dependent manner by treatment with the peptide complex.

Accordingly, these results confirm that the peptide complex of the present invention inhibits the expression of apoptosis-promoting factors induced by AGE-BSA or MGO and promotes the expression of anti-apoptotic factors in HaCaT keratinocytes and NIH3T3 fibroblasts, thereby suppressing cell apoptosis and improving skin cell damage.

### 3. Observation of effect for inhibiting expression of MMPs

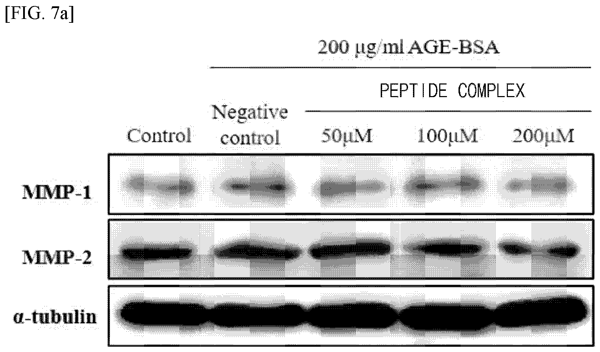

Matrix metalloproteinases (MMPs) are proteolytic enzymes that specifically act on collagen, and MMP-1 and MMP-2 degrade collagen, thereby reducing the elasticity of skin tissue and contributing to wrinkle formation. Accordingly, it was examined whether treatment of skin cells with the peptide complex of the present invention reduced the expression levels of MMPs, and the results are shown in FIGS. 7A, 7B, 8A, 8B, 9A, and 9B.

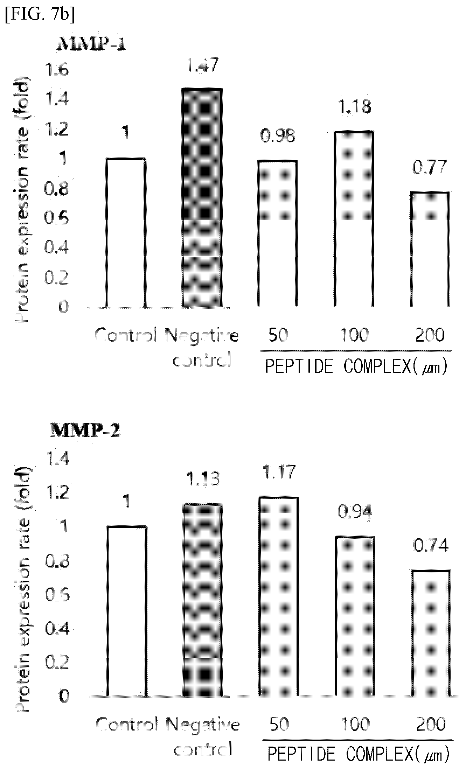

First, in HaCaT keratinocytes, the expression levels of MMP-1 and MMP-2 induced by AGE-BSA treatment and changes in the expression levels of MMP-1 and MMP-2 upon treatment with the peptide complex were examined, and the results are shown in FIGS. 7A and 7B. As shown in FIGS. 7A and 7B, the expression levels of MMP-1 and MMP-2 were increased by AGE-BSA treatment in a negative control group. However, as shown in the experimental groups, treatment with the peptide complex reduced the protein expression levels of MMP-1 and MMP-2.

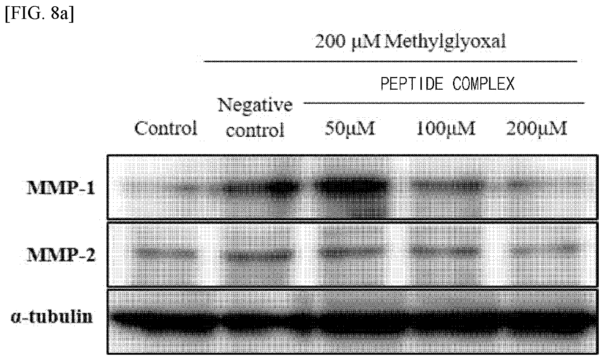

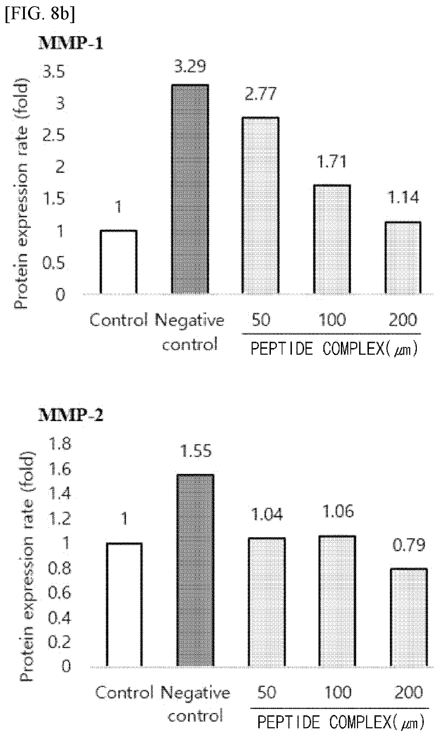

In addition, in HaCaT keratinocytes, the expression levels of MMP-1 and MMP-2 increased by MGO treatment and changes in the expression levels of MMP-1 and MMP-2 upon treatment with the peptide complex were examined, and the results are shown in FIGS. 8A and 8B. As shown in FIGS. 8A and 8B, the expression levels of MMP-1 and MMP-2 were increased by MGO treatment in a negative control group. However, as shown in the experimental groups, treatment with the peptide complex reduced the protein expression levels of MMP-1 and MMP-2 as compared to the negative control group.

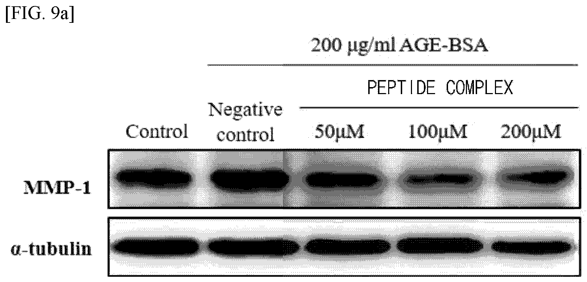

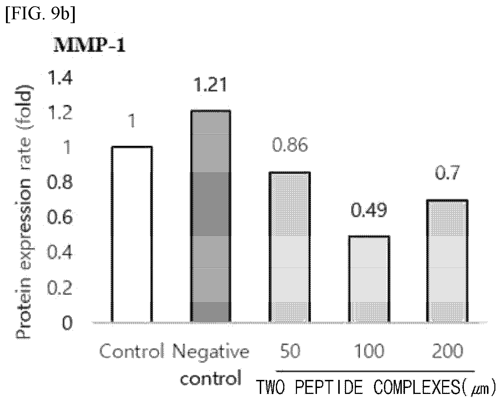

In NIH3T3 fibroblasts, the expression level of MMP-1 increased by AGE-BSA treatment and changes in the expression level of MMP-1 upon treatment with the peptide complex were examined, and the results are shown in FIGS. 9A and 9B. As shown in FIGS. 9A and 9B, the expression level of MMP-1 was increased by AGE-BSA treatment in a negative control group. However, as shown in the experimental groups, treatment with the peptide complex reduced the protein expression level of MMP-1 as compared to the negative control group.

Accordingly, these results demonstrate that the peptide complex of the present invention inhibits the expression of MMP-1 and MMP-2 increased by AGE-BSA or MGO treatment in HaCaT keratinocytes and NIH3T3 fibroblasts. Through this inhibition, the peptide complex reduces collagen degradation, enhances the elasticity of skin tissue, and prevents wrinkle formation, thereby improving skin damage.

### 4. Observation of effect for inhibiting expression of RAGE.

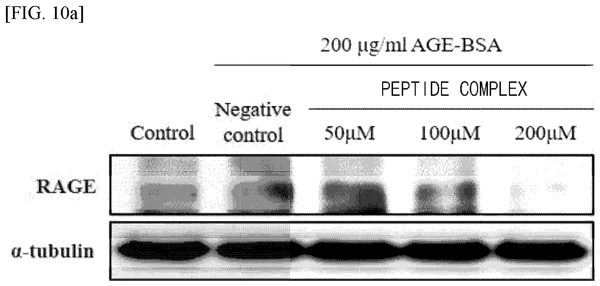

Receptor for Advanced Glycation End Products (RAGE) is a transmembrane receptor protein present on the cell surface that can bind advanced glycation end products (AGEs). When AGEs bind to RAGE in skin cells such as keratinocytes and fibroblasts, it is known that skin cell damage may be induced through the generation of reactive oxygen species (ROS), induction of inflammatory responses, and activation of apoptosis signaling pathways. Accordingly, it was examined whether treatment of skin cells with the peptide complex of the present invention reduced the expression level of RAGE, and the results are shown in FIGS. 10A, 10B, 11A, 11B, 12A, and 12B.

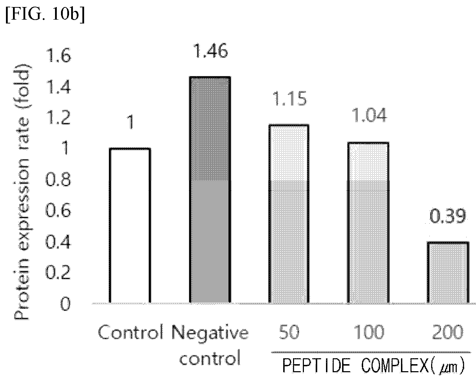

First, in HaCaT keratinocytes, the expression level of RAGE induced by AGE-BSA treatment and changes in the expression level of RAGE upon treatment with the peptide complex were examined. As shown in FIGS. 10A and 10B, the expression level of RAGE was increased by AGE-BSA treatment in a negative control group. However, as shown in the experimental groups, treatment with the peptide complex reduced the protein expression level of RAGE in a dose-dependent manner.

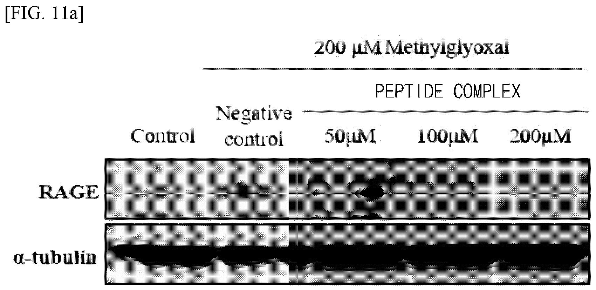

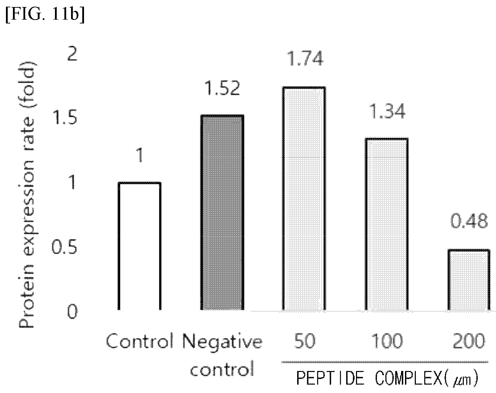

In addition, in HaCaT keratinocytes, the expression level of RAGE induced by MGO treatment and changes in the expression level of RAGE upon treatment with the peptide complex were examined. As shown in FIGS. 11A and 11B, the expression level of RAGE was increased by MGO treatment in a negative control group. However, as shown in the experimental groups, treatment with the peptide complex reduced the protein expression level of RAGE as compared to the negative control group at peptide complex concentrations of 100 µM or higher.

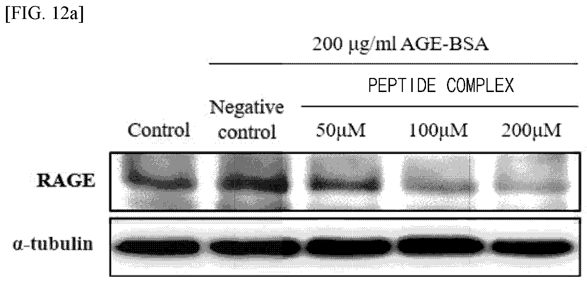

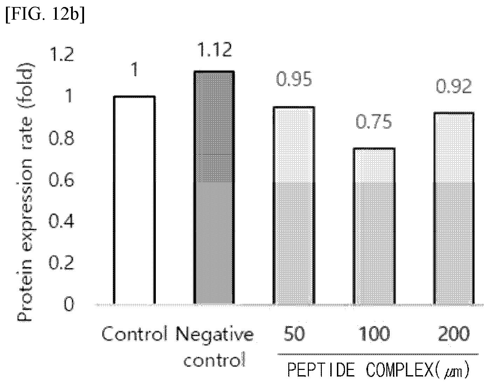

In NIH3T3 fibroblasts, the expression level of RAGE induced by AGE-BSA treatment and changes in the expression level of RAGE upon treatment with the peptide complex were examined. As shown in FIGS. 12A and 12B, the expression level of RAGE was increased by AGE-BSA treatment in a negative control group. However, as shown in the experimental groups, treatment with the peptide complex reduced the expression level of RAGE as compared to the negative control group.

Accordingly, these results demonstrate that the increased expression level of RAGE induced by AGE-BSA or MGO in HaCaT keratinocytes and NIH3T3 fibroblasts was reduced by treatment with the peptide complex of the present invention. Through this reduction, the peptide complex decreases the binding of AGEs, inhibits ROS generation, inflammatory responses, and activation of apoptosis signaling pathways, thereby improving skin damage.

### Experimental example 3: Evaluation of glyoxalase-1 expression levels for inhibiting the formation of advanced glycation end products

Reactive dicarbonyl compounds such as glyoxal, methylglyoxal, and 3-deoxyglucosone can be crosslinked with proteins to generate advanced glycation end products (AGEs). Glyoxalase-1 is a detoxifying enzyme that converts methylglyoxal into lactate, thereby eliminating methylglyoxal.

The effect of the peptide complex prepared in Preparation Example 1 on the expression level of glyoxalase-1 in HaCaT keratinocytes was evaluated by RT-PCR. First, human HaCaT keratinocytes were seeded in 6-well plates at a density of 2 × 10⁵ cells per well and cultured for 24 hours. Thereafter, the cells were pretreated with the peptide complex prepared in Preparation Example 1 at concentrations of 50 µM, 100 µM, and 200 µM for 1 hour. The pretreated cells were treated with 200 µM of methylglyoxal (MGO) and cultured in a CO₂ incubator at 37°C for 24 hours. After treatment, the cells were washed twice with PBS, and total RNA was isolated using easy-BLUETM (iNtRON, Cat. No.: 17061, Korea). The isolated total RNA was quantified, and cDNA was synthesized using an RT kit (enzynomics, Cat. No.: RT200, Korea). PCR was then performed using a PCR kit (enzynomics, Cat. No.: P581T, Korea). The primers used are shown in Table 4 below.

**[Table 4]**

| Cell line | Primer | Sequence (5'-> 3') | Sequence No. |
|---|---|---|---|
| HaCaT | Glyoxalase-1 Forward | GGTCCCGTCGTCTGTGATAC | 17 |
| | Glyoxalase-1 Reverse | TAGCTTTTCTGGAGAGCGCC | 18 |

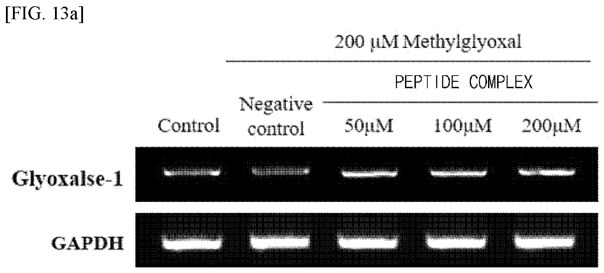

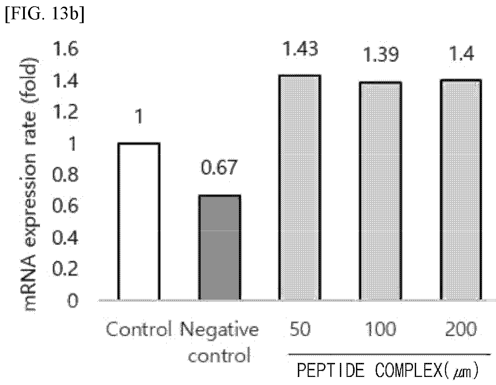

As a result, as shown in FIGS. 13A and 13B, the expression level of glyoxalase-1 was decreased by MGO treatment in a negative control group. However, as shown in the experimental groups, treatment with the peptide complex increased the expression level of glyoxalase-1. Accordingly, the peptide complex increased the expression of glyoxalase-1, which had been reduced by MGO treatment, thereby enabling the degradation of methylglyoxal, which is a precursor of AGEs. Through this mechanism, the formation of AGEs was inhibited, and skin damage was improved.

Accordingly, these results demonstrate that treatment of skin cells with the peptide complex of the present invention reduces the expression of inflammatory response factors induced by AGEs, thereby exhibiting antiinflammatory effects, and decreases the expression of apoptosis-related factors, thereby suppressing cell apoptosis. In addition, the expression of collagen-degrading enzymes is reduced, thereby enhancing the elasticity of skin tissue and preventing wrinkle formation. Furthermore, treatment with the peptide complex reduces the expression of RAGE, which is a receptor for AGEs, thereby decreasing AGE binding and inhibiting ROS generation, inflammatory responses, and activation of apoptosis signaling pathways, resulting in improvement of skin damage. In addition, the peptide complex increases the expression level of glyoxalase-1, which is reduced by MGO treatment, thereby promoting degradation of methylglyoxal, a precursor of AGEs, and inhibiting the formation of AGEs, leading to improvement of skin damage.

As described above, an example of the present disclosure is provided for the illustrative purpose, but the scope of the present disclosure is not limited to a specific example, a specific preparation example, and a specific experimental example. In other words, the present disclosure is modified appropriately within the scope of following claims of the present disclosure by those skilled in the art to which the present disclosure pertains.

## Claims

1. A composition for improving a skin condition, comprising as an active ingredient a peptide complex comprising: (i) a peptide comprising the amino acid sequence of SEQ ID NO. 1; and (ii) a peptide comprising an amino acid sequence of SEQ ID NO. 2.

2. The composition of claim 1, wherein the improving of the skin condition is at least one selected from the group consisting of alleviation of skin inflammation, improvement of skin aging, promotion of skin regeneration, improvement of skin elasticity, alleviation of skin wrinkles, improvement of skin barriers, and improvement of skin damage.

3. The composition of claim 1, wherein the composition exhibits at least one activity selected from the following (a) to (e),
(a) inhibition of expression of TNF-α, IL-1 β, or IL-6, or inhibition of expression of COX-2;
(b) inhibition expression of cleaved PARP-1, cleaved caspase-3, cleaved caspase-9 or Bax, or increase in expression of Bcl-2;
(c) inhibition of expression of MMP-1 or MMP-2;
(d) inhibition of expression of Receptor Advanced Glycation End Products (RAGE); and
(e) increase in expression of glyoxalase-1.

4. The composition of claim 1, wherein the composition is a cosmetic composition.

5. The composition of claim 4, wherein the cosmetic composition has any one formulation selected from the group consisting of a solution, a suspension, an emulsion, a paste, a gel, a cream, a lotion, a powder, a soap, a surfactant-containing cleansing, oil, a powder foundation, an emulsion foundation, a wax foundation, and a spray.

6. A pharmaceutical composition for treating or preventing skin inflammation or skin disease, comprising a therapeutic effect amount of a peptide complex comprising: (i) a peptide comprising an amino acid sequence of SEQ ID NO. 1; and (ii) a peptide comprising an amino acid sequence of SEQ ID NO. 2.

7. The composition of claim 6, wherein the skin disease is selected from the group comprising bacterial skin infections, skin wounds, diabetic skin wounds, diabetic dermatopathy, diabetic foot disease, hyperpigmentation disorders, photo-induced hyperpigmentation disorders, psoriasis, and systemic lupus erythematosus.

8. A functional food composition for improving a skin condition, comprising as an active ingredient a peptide complex comprising: (i) a peptide comprising an amino acid sequence of SEQ ID NO. 1; and (ii) a peptide comprising an amino acid sequence of SEQ ID NO. 2.

9. The composition of claim 8, wherein the improving of the skin condition is at least one selected from the group comprising alleviation of skin inflammation, improvement of skin aging, promotion of skin regeneration, improvement of skin elasticity, alleviation of skin wrinkles, improvement of skin barriers, and improvement of skin damage.
